# EUROPEAN PATENT SPECIFICATION

(11) **EP 0 248 647 B1**
(45) Date of publication and mention of the grant of the patent: **13.01.1993**
(21) Application number: 87304900.1
(22) Date of filing: 03.06.1987
(51) Int. Cl.: C12N 15/58, C12N 15/85, A61K 37/02

(54) **Expression vectors containing the heat shock regulatory DNA sequences from a heat shock protein 83 (hsp83) gene and inducible expression by the use of the vectors**
Expressionsvektoren, die die Hitzeschock-Regulator-DNA-Sequenzen eines Hitzeschock-Protein-83-(hsp 83)-Gens enthalten und induzierbare Expression unter Verwendung der Vektoren
Vecteurs d'expression contenant des séquences d'ADN régulateur de choc de chaleur d'un gène de protéine 83 de choc de chaleur (hsp 83) et expression induisible par utilisation de ces vecteurs

(30) Priority: 03.06.1986 JP 127172/86
(43) Date of publication of application: 09.12.1987
(73) Proprietor: MITSUI TOATSU CHEMICALS, Inc., Chiyoda-Ku Tokyo 100 (JP)
(72) Inventor: Tomioka, Noboru, Mobara-shi Chiba-ken (JP); Omae, Fumio Mitsui Toatsu Kosugaya Apaato 4-1-7, Yokohama-shi Kanagawa-ken (JP); Mine, Eriko, Tokyo (JP); Ishii, Tomoko, Chiba-ken (JP)
(74) Representative: Holdcroft, James Gerald, Dr.

(56) References cited:
- EP-A- 0 118 393
- WO-A-84/04535
- PROCEEDINGS OF THE NATL. ACADEMY OF SCIENCES USA, vol. 78, no. 6, June 1981, Baltimore, MD (US); R.HOLMGREN et al., pp. 3775-3778
- M.J.SCHLESINGER et al., "Heat Shock from Bacteria to Man", 1982, Cold Spring Harbor Lab., New York, NY (US); J.LIS et al., pp. 57-62
- NUCLEIC ACIDS RESEARCH, vol. 11, no. 20, 1983, Oxford (GB); R.W.HACKETT et al., pp. 7011-7030
- NATURE, vol. 311, no. 5981, 06 September 1984, New York-London; C.WU, pp. 81-84

## Description

### Background of the Invention

### 1. Field of the Invention

The present invention relates to the technologies to produce desired foreign gene products such as physiological active substances, etc., by use of the heat shock regulatory DNA sequences from a Drosophila melanogaster heat shock protein 83 (hsp83) gene.

### 2. Description of the prior art

Related technology has been developed using heat shock regulatory DNA sequences from Drosophila melanogaster heat shock protein 70 (hsp70) genes. Several foreign genes have been expressed under the control of the hsp70 regulatory sequences (Corces, V. et al., 1982, in Heat Shock from Bacteria to Man, Eds., Schlesinger, M.J., et al., Cold Spring Harbor Laboratory, p27; Pelham, H.R.B., 1982, Cell, 30:517; Pelham, H.R.B. and Bienz, M., 1982, EMBO Journal, 1:1473; Bromley, P. and Voellmy, R., 1984, European Patent Publication No. 0118393A2; Kingston, R.E. et al., 1984, Nature, 312:280) but the yield of mature gene products has been quite limited.

For example, an influenza haemagglutinin protein gene has been expressed using the hsp70 heat shock regulatory DNA sequences and the expression data have shown ambiguous manners of expression in monkey COS cells or Xenopus oocyte cells by Bromley and Voellmy (Bromley, P. and Voellmy, R., 1984, European Patent Publication No. 0118393A2). In most of their expression experiments, haemagglutinin was detected at normal temperature without a heat shock treatment. In some of their experiments, heat shock temperature (43°C) resulted inhibitory effect on the haemogglutinin production. The amounts of haemagglutinin produced have been detected by immuno-precipitation and following polyacrylamide gel electrophoresis. Generally, the immuno-precipitation method is employed to detect the gene product whose content is very small, therefore the amounts of haemagglutinin expressed under the control of the hsp70 heat shock regulatory sequences in their experiments are not expected to be large.

By using hsp70 heat shock regulatory sequences, a herpes simplex virus thymidine kinase (HSV-tk) gene and a human growth hormone gene have been expressed in mouse L cells (Corces, V. et al., 1982, in Heat Shock from Bacteria to Man, Eds., Schlesinger, M.J. et al., Cold Spring Harbor Laboratory, p27) and the HSV-tk gene in the monkey COS cells (Pelham, H.R.B. and Bienz, M., 1982, EMBO Journal, 1:1473). In these expression experiments, the levels of these foreign gene expression have been examined by the measurements of mRNA contents probably because the levels have been not highly enough to detect translational products One of the reasons for these low expression has been considered that the hsp70 promoter is extremely weak one (Kingston, R.E. et al., 1984, Nature, 312:280).

In addition, as explained later, the present inventors tried to express a tissue plasminogen activator (tPA) gene which was originated from human normal cells using the heat shock regulatory DNA sequences from the Drosophila hsp70 gene but the expression experiment resulted in almost negligible amounts of tPA production.

Other heat shock regulatory DNA sequences from Drosophira melanogaster hsp83 have been analyzed (Holmgren, R. et al., 1981, Proc. Natl. Acad. Sci., 78:3775; Wu, C., 1984, Nature, 309:229; Wu, C., 1984, Nature, 311:81) and it is known that a part of the heat shock regulatory DNA sequence has almost perfect dyad symmetry structure and that this regulatory DNA sequence binds with a putative heat shock activator protein.

Expressions of foreign genes using hsp83 regulatory DNA sequences have been attempted, but no desirable results have yet been attained.

For example, although the Drosophila hsp83 gene itself was expressed in yeast cells (Lis, J. et al., 1982, in Heat Shock from Bacteria to Man, Eds., Schlesinger, M.J. et al., p57), transcriptional activity of the Drosophila hsp83 heat shock regulatory DNA sequences in transformed animal cells has not been successfully examined as far as the present inventors know.

Analysis of the Drosophila hsp83 heat shock regulatory region has been performed (Wu, C., 1984, Nature, 309:229; Wu, C., 1984, Nature, 311:81). A putative heat shock activator protein (HAP) binding site of the hsp83 gene has been shown by analyzing exonuclease resistant sites. A DNA sequence of the HAP binding site has been shown to contain a 28 base sequence with almost perfect dyad symmetry. The sequence with dyad symmetry may cause a stem structure as shown below. The inventors do not find any DNA sequence with good dyad symmetry around the hsp70 heat shock consensus sequence which was predicted by Pelham (Pelham, H.R.B., 1982, Cell, 30:517). An example of the possible stem structure for the hsp70 sequence is shown below. Stars indicate location of the consensus sequence.

G T
*A-T*
*A-T*
*G-C*
A-T
*T-A*
*C-G*
T-A
C-G
C   A
G-C
A-T
A-T
G-C
ATCCA   CAGT

hsp83

T G
*A-T*
*A-T*
*G-C*
C-G
T   C
*C-G*
C   A
G   A
C   A
G   A
C-G
G   A
A   G
GAGAG   CGCC

hsp70

Although Wu has been reported that the hsp83 gene binds with the putative HAP stronger than the hsp70 gene does (Wu, C., 1984, Nature, 311:81), other reports have pointed out that the level of hsp83 gene expression is lower than that of hsp70 gene expression at heat shock temperature and that the hsp83 gene is slowly expressed at normal growth temperature in some Drosophila cells. (Hackett, R.W. and Lis. J.T., 1983, Nucl. Acids Res., 11:7011; Ashburner, M. and Bonner, J.J., 1979, Cell, 17:241).

Although hsp83 heat shock regulatory DNA sequences may be useful in the fields of gene-technological production of foreign genes, effective uses of them, however, have not yet been proven.

### Summary of the Invention

The present inventors have suspected that problems might be included in the hsp70 heat shock regulatory DNA sequences, when one intends to employ the hsp70 sequences in order to express foreign genes in eukaryotic cells after induction. To resolve the difficulty, the inventors introduced into animal cells the heat shock regulatory DNA sequences from the Drosophila hsp83 gene into foreign gene expression vectors and obtained transformants by use of these vectors. The resulting Resulted transformants produced certain amounts of the gene product only after heat shock induction and thus the invention has been completed.

Although the strong and regulatory expression in the construction of the present invention had not been expected from the prior art as noted above, the present inventors, however, found unexpectedly the strong and regulatory expression of a foreign gene in animal cells by introducing the heat shock regulatory DNA sequences from Drosophila hsp83 gene into the construction described below. The results of the present inventors showed that the foreign gene could be expressed strongly in the transformed cells only after heat shock induction.

An object of the present invention is to provide reproducible technologies for obtaining stronger expression of the foreign gene encoded a physiologically active substance and the like in eukaryotic cells, especially in animal cells.

The present invention is characterized by the use of the heat shock regulatory DNA sequences from a Drosophila melanogaster heat shock protein 83 (hsp83) gene to perform the object mentioned above. The modified sequences of the regulatory sequences may be used instead of the hsp83 sequences. The hsp83 heat shock regulatory DNA sequences and other DNA sequences necessary for translation are linked functionally to the foreign structural genes which encode physiologically active substances, etc., and thus expression vectors are constructed.

Eukaryotic cells can be transformed with the expression vectors. The transformed cells are grown and the production of the desired foreign gene products by the cells are induced by the heat shock treatment.

According to the present invention, foreign genes encoding for physiologically active substances, etc. can be expressed after induction in eukaryotic cells, especially in animal cells, and the physiologically active substances, etc. can be easily recovered from the cells or culture media. According to the inducible expression described in the present invention, expression of a foreign gene is usually repressed and expression can be turned on when necessary by heat shock treatment. The present invention also enables one to obtain transformed cells which possess the potential ability to produce a physiologically active substance in toxic amounts for growth of the host cells.

### Brief Description of the Drawings

Figure 1 is a schematic representation of the procedure used to obtain the approximately 200 bp HindIII fragment containing the hsp83 heat shock regulatory DNA sequences.

Figures 2A & 2B shows the construction procedure of plasmid pSVtPA.

Figure 3 shows the nucleotide sequence of one strand of the PvuII-BglII fragment in the plasmid pSVtPA.

Figure 4 shows the construction procedure of plasmid pHStPA.

Figure 5 shows the nucleotide sequence of one strand of the DNA fragment containing two HindIII sites and a BglII site in the plasmid pHStPA.

Figure 6 shows the construction procedure of plasmid pHStPA-tk.

Figure 7 shows northern blot analysis to visualize the amounts of tPA mRNA produced by the clone #P48 before and after heat shock induction.

Figure 8 shows the results of the fibrin-agar assay after separating tPA molecules with polyacrylamide gel. The tPA molecule which was produced by clone #P48 were analyzed before and after heat shock treatment.

### Detailed Description of Preferred Embodiments

The present invention includes the construction of the expression vectors using the heat shock regulatory DNA sequences from the Drosophila melanogaster approximately 83k daltons heat shock protein (hsp83) gene or the modified sequences of these regulatory sequences, methods for obtaining transformants using these expression vectors, methods for inducible expression of the desired foreign genes and methods for recovering their gene products.

As used herein "the heat shock regulatory DNA sequences from the Drosophila melanogaster approximately 83k daltons heat shock protein (hsp83) gene" means the DNA sequences responsible for the hsp83 gene expression. These DNA sequences comprise the TATA box sequence upstream of the hsp83 structural region shown by Holmgren et al. (Holmgren, R. et al., 1981, Proc. Natl. Acad. Sci., 78:3775), the regulatory DNA sequence upstream of the TATA box sequence shown by Wu (Wu, C., 1984, Nature, 309:229) and other unidentified regulatory DNA sequence(s) which may participate in transcriptional and/or translational regulation of the hsp83 gene.

Drosophila melanogaster hsp83 has been reported variously as hsp80, -81, -82 and -83. Recently, hsp83 was referred to as hsp82 because the molecular weight of the hsp83 was calculated to be 81,853 from the complete nucleotide sequence data of the gene (Wu, C., 1984, Nature, 311:81).

"Modified sequences" refers to the sequences which are modified from the original sequences by base-substitution, deletion, insertion and replacement without decrement or with increment of the efficiency of the heat shock regulatory expression which is the aim of the present invention.

Heat shock proteins (hsp) are known to be highly conserved among eukaryotes. The higher molecular weight hsp, whose molecular weights are from 80k to 90k daltons, have been identified not only in Drosophila but also in mouse, human and other eukaryotic cells (See Heat Shock from Bacteria to Man, 1982, Eds., Schlesinger, M.J. et al; Lai, B.-T., et al., 1984, Mol. Cell. Biol., 4:2802). Therefore, if the heat shock regulatory DNA sequences were found in the eukaryotic high molecular weight hsp genes and these sequences were identical or similar to those from the Drosophila hsp83 gene, the sequences might be included in the "modified sequences".

The heat shock regulatory DNA sequences of the Drosophila hsp83 gene can be isolated by enzymatic or physical methods from the hsp83 gene which was already cloned by Holmgren et al. (Holmgen, R. et al., 1981, Proc. Natl. Acad. Sci., 78:3775). The DNA sequences can be prepared by the DNA synthesis method (Itakura, K. et al., 1984, Ann. Rev. Biochem., 53:323) because the partial and even the entire DNA sequence of the hsp83 gene has been published (Holmgren, R. et al., 1981, Proc. Natl. Acad. Sci. 78:3775; Hackett, R.W. and Lis, J.T., 1983, Nucl. Acids Res., 11:7011).

The inventors obtained conveniently the approximately 190 base pair DNA fragment containing the Drosophila hsp83 heat shock regulatory DNA sequences using restriction enzymes.

"Modified sequences" of the Drosophila hsp83 heat shock regulatory DNA sequences can be prepared by the DNA synthesis method. If this method was employed, one can easily design and then obtain the modified sequences by base exchange, deletion, insertion, transposition and so on. Other ways to obtain the modified sequences are enzymatic or physical isolation of the sequences from the eukaryotic higher molecular hsp genes which are related to the Drosophila hsp83 gene. Point mutation or other mutation methods containing in vitro mutagenesis will provide the method to give the modified sequences of the hsp83 sequences.

"Expression vector" means vectors which are capable of expressing certain genes contained therein, and its true form is DNA. In general, expression vectors are treated in the form of "plasmids" which refer to circular double stranded DNA loops. The DNA constructs of the expression vector will include at least one DNA fragment containing the heat shock regulatory DNA sequences, a foreign structural gene coding for the desired substance such as physiologically active substances, at least one DNA fragment containing a terminator and a polyadenylation signal. All DNA fragments will be connected to each other in the vector to give transcriptional and/or translational control for the structural gene. The functional unit to give transcriptional and/or translational control for the structural genes may be called an "expression unit". The DNA fragment containing the terminator and the polyadenylation signal can be derived from the foreign gene itself or any other suitable gene. The "expression unit" will contain untranslated 5'-flanking and/or untranslated 3'-flanking DNA sequences of the desired foreign gene and in some cases intervening sequences of the foreign gene. The "expression unit" may also contain the DNA sequences which do not inhibit gene expression upstream and/or downstream of the control sequences. Namely, flanking DNA sequences of the heat shock regulatory sequences, the terminator sequences and linker DNAs may be contained in the "expression unit". The "expression unit" will be introduced into expression vectors.

"Expression vectors" must be replicable in the host organisms either as episomes or as an integral part of the chromosomal DNA. Autonomously replicating sequences, which are derived from simian virus 40 (SV40), bovine papilloma virus, etc., cause expression vectors replicable in host cells as episomes. Episomes cause a higher but unstable copy number of the desired foreign gene in host cells.

Integration of the expression vector in the chromosomal DNA of host cells results in a usually low but stable copy number because the integrated genes are replicable in accordance with replication host chromosomes. The integration method is preferable when stable expression is desired.

In the present invention, the selection markers are employed for the "expression vectors" to select transformants. The selection markers effective for animal cells are described later in the explanation of host cells.

In the present invention, it is advantageous to provide a prokaryotic replication system and a prokaryotic selection marker to allow for cloning of the expression vector in a bacterial host. The prokaryotic replication origin such as the plasmid pBR322 origin and the prokaryotic selection marker such as the ampicillin resistance gene are introduced into the expression vector and this enables one to select the transformed bacterial cells with which large amounts of the vector plasmid DNA can be recovered.

"Host cells" refers to cells which are used for transformation by the introduction of the expression vectors. The host cells will be selected as suitable for the function of the expression vector. The host cells must have the ability to transcribe and translate the expression unit for the desired foreign gene.

In some cases, the host cells need the ability to provide modifications for the gene products. In another cases, the host cells need the ability to translocate and/or secrete the gene products.

Any method by which DNA is incorporated into the host cells can be employed to introduce the expression vector plasmid DNA into the host cells. The calcium chloride method (Mandel, M. and Higa, A., 1970, J. Mol. Biol., 53:154) is widely used for prokaryotic host cells and the calcium phosphate method (Wigler, M. et al., 1979, Proc. Natl. Acad. Sci., 76:1373) for animal host cells. Both methods will be effective for the present invention.

Dominantly action markers such as a neomycin resistance (Neo^{R}) gene (Colbere-Garapin, F. et al., 1981, J. Mol. Biol., 150:1), a bacterial xanthine guanine phosphoribosil transferase (XGPRT) gene (Mulligan, R.C. and Berg, P., 1981, Proc. Natl. Acad. Sci., 78:2072) are useful in selecting for transformed animal cells. A thymidine kinase (tk) gene (Wigler, M. et al., 1977, Cell, 11:223) or a dihydroforate reductase (DHFR) gene (Subramani, S. et al., 1981, Mol. Cell. Biol., 1:854) is also useful in selecting for transformed animal cells. The deficient cells for the tk or the DHFR gene are preferable for transformation experiments.

The co-amplification method reported by Ringold et al. (Ringold, G. et al., 1981, J. Mol. Appl. Gent., 1:165) may enable one to amplify the desired foreign gene in concert with the DHFR gene amplification. The method may be useful and can be easily applied in the present invention to increase the copy number of the desired gene. High copy number may result in stronger expression of the desired gene in the present invention.

As used herein "physiologically active substances" means a variety of polypeptides or the associated form of these polypeptides such as enzymes, hormones, clotting factors, lymphokines, antigens of virus, other antigens, and so on. Especially when the polypeptides are derived from eukaryotic cells, many polypeptides are modified by proteolitic cleavage, glycosylation, acetylation, phosphorylation, processing for maturation, etc. with special enzymes located in the eukaryotic cells. Some polypeptides are folded into complicated higher structures by the aid of S-S bonds or other molecular interactions. The post-translational modifications and higher structures may not occur correctly in prokaryotic cells.

When the physiologically active substance is a human tissue plasminogen activator (tPA) whose physiological function is to dissolve fibrin clot, the translated product of a tPA gene is cleaved at the end of the N-terminal leader amino acid sequence and subjected to glycosylation, the resulting mature tPA is folded into higher structures by the aid of S-S bonds in the cells and finally the mature product is secreted outside the cells (Rijken, D.C. and Collen, D., 1981, J. Biol. Chem., 256:7035; Pennica, D. et al., 1983, Nature, 301:214).

Human tPA was selected as an example of the physiologically active substance in the present invention because of its nature. The present invention is, of course, not restricted by the example.

Human tPA genes may be available as genomic clones obtained from human chromosomal DNA (Ny, T. et al., 1984, Proc. Natl. Acad. Sci., 81:5355; Browne, M.J., et al., 1985, Gene, 33:279; Fisher, R. et al., 1985, J. Biol. Chem., 260:11223) or as cDNA clones drived from melanoma mRNA (Pennica, D. et al., 1983, Nature, 301:214; Goeddel, D.V. et al., 1983, UK Patent Application No. GB2119804A) and normal cell mRNA (European Patent Application No. 86309250.8).

Using one of the human tPA genes as an example of the foreign physiologically active substance gene, a general description of the preferred embodiments are presented here and in the experimental section hereinafter to demonstrate the advantages and benefits of the present invention.

The DNA fragment containing the Drosophila hsp83 heat shock regulatory DNA sequences was isolated from a Drosophila hsp83 clone. The fragment was joined functionally to the tPA structure gene which was derived from human normal cells and placed in the expression vector. The DNA fragment containing the sequences responsible for termination of transcription and polyadenilation were inserted to give satisfactory expression of the tPA gene in the expression vector.

The replication origin which was required for replication in E. coli cells, the selection marker effective in E. coli cells and other DNA sequences required for the tPA gene expression were introduced into the expression vector.

Mammalian cultured cells, widely used for foreign gene expression experiments, were employed as host cells.

The selection marker gene such as a tk gene was introduced to the expression vector and the insertion of the marker gene resulted in convenient selection of the transformants. Tk deficient cells were employed as host cells in the case of tk selection.

The host cells were transformed with the expression vector DNA and the resulting transformants were subjected to evaluation of tPA production under the conditions of heat shock induction. The amount of tPA accumulated in the culture medium was measured by the fibrin clot lysis assay. When the heat shock induction was performed prior to measurement of tPA, certain amounts of tPA were detected under the control of the hsp83 heat shock regulatory DNA sequences although no detectable tPA was produced in the absence of heat shock induction.

In a comparative experiment, the DNA fragment including the Drosophila hsp70 heat shock regulatory DNA sequences (See Pelham, H.R.B., 1982, Cell, 30:517) was isolated from the subclone B8 (Craig, E.A. et al., 1979, Cell, 16:575; Ingolia, T.D. et al., 1980, Cell, 21:669) with restriction enzymes. The DNA fragment containing hsp70 heat shock regulatory DNA sequences was inserted in the proper site of the tPA expression vector in the proper orientation. The construction of the expression vectors and the other methods and procedurs to obtain tPA gene expression were followed as the hsp83 experiments mentioned in the section of examples.

Only trace amounts of tPA were detected under the control of the hsp70 heat shock regulatory DNA sequences when heat shock induction was performed prior to the measurement of tPA and no tPA activity was detected without heat shock induction. The results indicated that the hsp83 regulatory DNA sequence was preferable for inducible expression of foreign genes.

Production of tPA under the control of the hsp83 regulatory DNA sequence was interpreted to be relatively high because the amounts of tPA produced by the hsp83 regulatory sequences were comparable to that of the constitutive expression under the control of the SV40 early promoter.

The following examples are intended to illustrate but not to limit the invention.

Moreover, it is to be understood that, where no specific procedures and operating conditions are indicated in the following examples, ordinary procedures and operating conditions apparent to those skilled in the art were suitably chosen and employed.

### Example 1

### [Isolation of the DNA Fragment Containing Drosophila hsp83 Heat Shock Regulatory Sequences and Construction of Expression Vectors Using the Fragment]

### 1-A Preparation of the 200 bp HindIII Fragment Containing Drosophila hsp83 Heat Shock Regulatory Sequences

A BamHI-SalI subclone aDm4.46 (Hackett, R.W. and Lis, J.T., 1983, Nucl. Acids Res., 11:7011) containing the 5'-flanking region of a Drosophila melanogaster hsp83 gene was digested with the restriction endonucleases EcoRI (Bethesda Research Laboratories INC; abbreviated as BRL) and Xhol (BRL), and the resulting DNA fragments were fractionated by agarose gel electrophoresis. The 760 base pairs (bp) EcoRI-XhoI fragment was recovered by electroelution (See Molecular Cloning, A Laboratory Manual, 1982, Eds., Maniatis, T. et al., Cold Spring Harbor Laboratory, p150). The unit "base pairs (bp)" represents the length of DNA and the numerical value shown in the examples hereinafter will provide only approximate information.

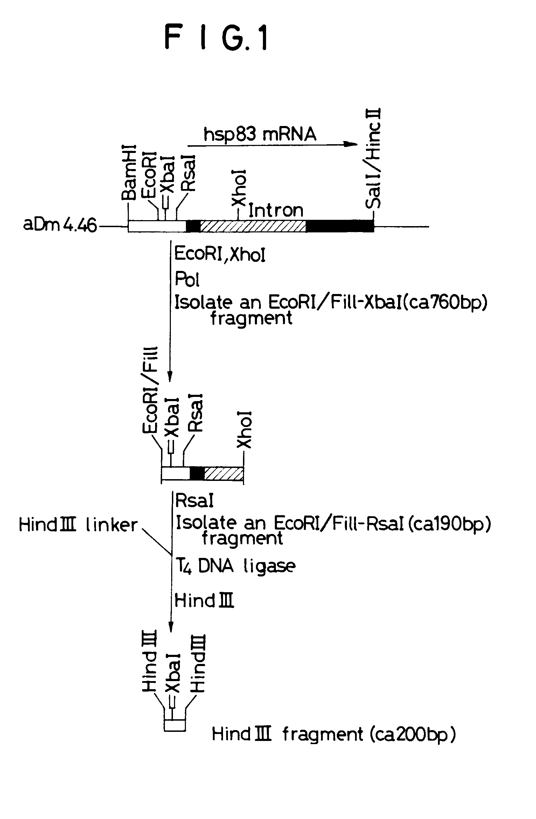

The 3'-cohesive ends of the 760 bp fragment were extended to blunt ends by filling in with Klenow DNA polymerase I. The 760 bp fragment with blunt ends was digested with the restriction endonuclease RsaI (New England Biolabs; abbreviated as NEB) and the resulting fragments were fractionated by agarose gel electrophoresis. A 190 bp EcoRI/fill-RsaI fragment was recovered by electroelution. A commercially available HindIII linker DNA (d5'-GCAAGCTTGC-3', BRL) was phosphorylated at its 5'-end with T4 polynucleotide kinase (BRL) as described in Molecular cloning, A Laboratory Manual, 1982, Eds., Maniatis, T. et al., p125. 450 ng of phosphorylated HindIII linker DNA and 630 ng of 190 bp EcoRI/fill-RsaI fragment were dissolved in 37 µl of the ligation buffer which was suggested by the supplier and ligated by adding 6 units of T4 DNA ligase (BRL) and followed by incubation at 12°C for 5 hours. The reaction was terminated by heating at 65°C for 5 minutes and then chilled quickly at 0°C. The resulting fragments were digested with HindIII (BRL), subjected to agarose gel electrophoresis and a 200 bp HindIII fragment (See figure 1) was electroeluted. The 200 bp HindIII fragment contains the hsp83 heat shock regulatory DNA sequences.

### 1-B Construction of Plasmid pSVtPA

Plasmid pSVM-dhfr (Lee, F. et al., 1981, Nature, 294:228) was digested with the restriction endonuclease BglII and filled in with Klenow DNA polymerase I (BRL) as described (See Molecular Cloning, A Laboratory Manual, 1982, Eds., Maniatis, T. et al., p113). Specifically, the 5'-cohesive ends of the resulting linear DNA were extended to blunt ends by filling in with Klenow DNA polymerase I. The thus obtained linear DNA fragment whose 5'-ends were blunt was digested with HindIII, dephosphorylated at its 5'-ends with bacterial alkaline phosphatase (BAP, BRL) as described (Ullrich, A. et al., 1977, Science, 196:1313) and subjected to agarose gel electrophoresis. The 4.4 kilo base pairs (kb) DNA fragment was electroeluted. One end of the fragment was HindIII cohesive end and other was blunt end.

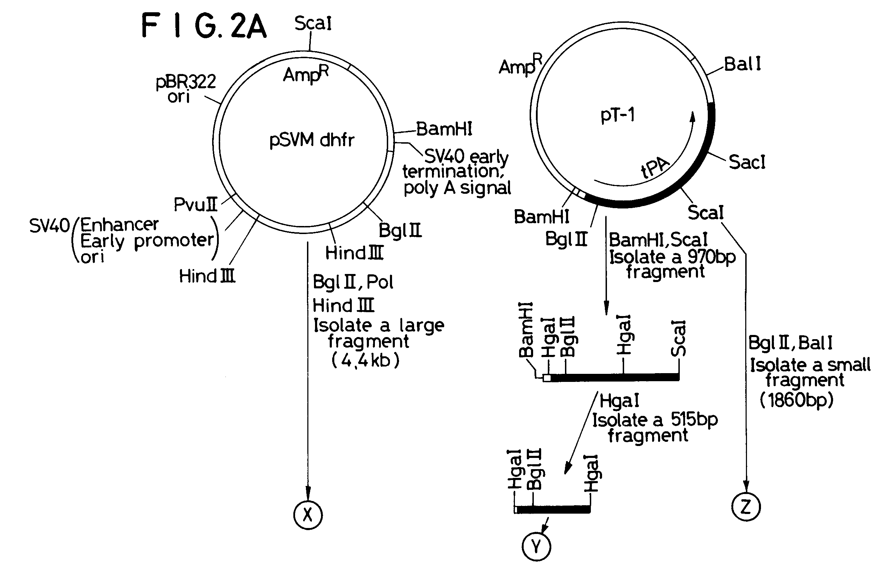

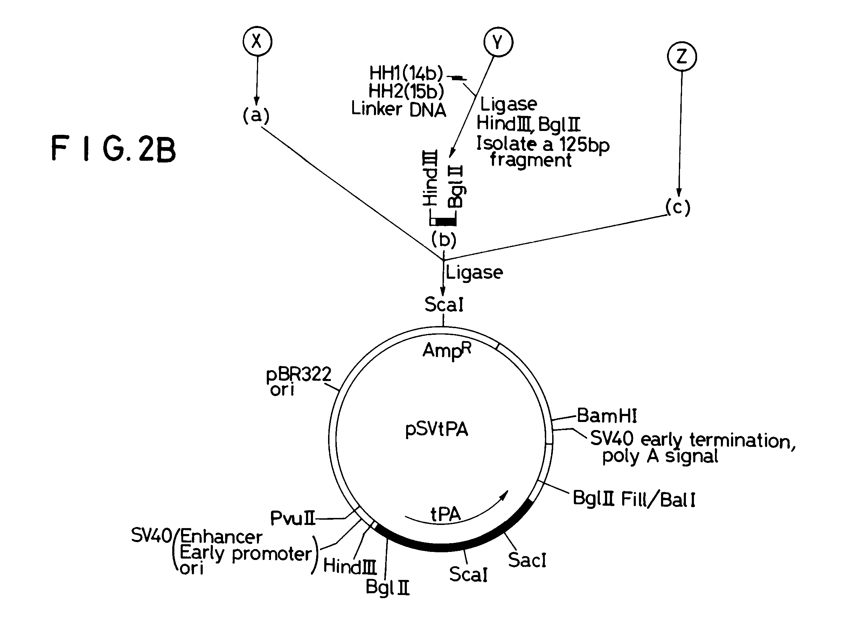

The 4.4 kb fragment contains an SV40 early promoter, and SV40 enhancer, and SV40 replication origin, a prokaryotic replication origin, an ampicillin resistance gene (Amp^{R}), SV40 early transcript termination and polyadenylation signals and other non-translating DNA fragments (Figure 2B-a).

Plasmid pT-1 (European Patent Application No. 86309250.8) contains a human tPA cDNA derived from normal cells (MTC-017). The entire tPA coding region was isolated from the plasmid as follows.

The plasmid DNA was digested with the restriction endonucleases BglII and BalI (NEB) and the digested DNA fragments were separated by agarose gel electrophoresis. A 1860 bp DNA fragment carrying the C-terminal amino acids coding region and the 3'-flanking region (See Fig. 2B-c) was isolated by electroelution. One end of the fragment was BglII cohesive and the other was blunt.

Separately, pT-1 was digested with the restriction endonucleases BamHI (BRL) and ScaI (NEB) and subjected to agarose gel electrophoresis and then a 970 bp DNA fragment was electroeluted. The fragment carried the N-terminal amino acids coding region and also the 5'-flanking region. The fragment was digested with the restriction endonuclease HgaI (NEB) and the digested DNA fragments were fractionated by agarose gel electrophoresis. The 515 bp DNA fragment carrying the N-terminal amino acids coding region and also the 7 bp 5'-flanking region was recovered by electroelution.

Two different oligodeoxinucleotides were synthesized by the solid-phase phosphotriester method (Itakura, K. et al., 1984, Ann. Rev. Biochem., 53:323). One of the oligodeoxinucleotide (5'-AGCTTACGCTGTGA-3') was designated as HH-1 and the other (5'-TTGCTTCACAGCGTA-3') was as HH-2. The double strand DNA obtained after annealing of the synthetic DNAs was comprised of one HindIII cohesive end and other cohesive end whose sequence was complementary to the HgaI end of the above mentioned 515 bp DNA fragment. Except for the HindIII sequence, the DNA sequence of the resulting double strand DNA was resulted identical to the sequence upstream of HgaI site in the 5'-flanking region of the tPA gene.

400 ng of HH-1 DNA, 400 ng of HH-2 DNA whose 5'-end was phosphorylated with T4 polynucleotide kinase and 600 ng of the 515 bp DNA fragment were dissolved in 20 µl of ligation buffer and ligated by adding 0.15 units of T4 DNA ligase and followed by incubation at 15°C for 1 hour. The ligated DNAs were digested with BglII and HindIII and the digested DNA fragments were fractionated by polyacrylamide gel electrophoresis (See Molecular Cloning, A Laboratory Manual, 1982, Eds., Maniatis, T. et al., p174). The 125 bp HindIII-BglII fragment containing the DNA sequence derived from the synthetic oligodeoxinucleotide (Fig. 2B-b) was electroeluted. The 125 bp fragment lacked phosphate at its HindIII 5'-end.

Plasmid pSVtPA was constructed by ligating the 4.4 kb DNA fragment (Fig. 2B-a), the 125 bp DNA fragment (Fig. 2B-b) and the 1860 bp DNA fragment (Fig. 2B-c) as follows:
150 ng of the 4.4 kb DNA fragment, 12 ng of the 125 bp DNA fragment and 200 ng of the 1860 bp DNA fragment were dissolved in 15 µl of the ligation buffer and ligated by adding 0.02 units of T4 DNA ligase and followed by incubation at 15°C for 1 hour. The reaction mixture was further incubated at 15°C for 1 hour after another 2 units of the ligase was added thereto and then the reaction was completed by incubating it at 65°C for 5 minutes and cooling it to 0°C.

The ligated DNAs were introduced into E. coli strain DH-1 (Hanahan, D., 1983, J. Mol. Biol., 166:557) by the calcium chloride method (Mandel, M. and Higa, A., 1970, J. Mol. Biol., 53:154). Transformants were selected for ampicillin resistance. Ampicillin resistant colonies were screened by the colony hybridization method (Grunstein, M. and Hogness, D.S., 1975, Proc. Natl. Acad. Sci., 72:3961). One of the probes used for screening was the HH-1 DNA which was labeled with [γ³²P]ATP (Amersham) and T4 polynucleotide Kinase (Probe 1). The 470 bp EcoRI fragment which encoded for tPA gene sequence was nick translated with [α³²P]CTP (Amerhsam) and nick translation kit (BRL) (Probe 2).

46 transformants were grown on two series of the nitrocellulose filters (#541; Whatman). The filters containing the transformants were treated with alkali and then neutralized, washed and dried in air. The filters were prehybridized at 55°C for 2 hours. The solution used for prehybridization was 180 mM Tris-HCl (pH 8.0), 6 mM EDTA, 0.9 M NaCl, 0.5% honidet-NP40 Noidet-P40 (NP-40;BRL), 5x Denhart's (Ficol 1 g, polyvinylpyrollidone 1 g and BSA 1 g in 1000 ml solution), 100 ug/ml of sonicated salmon sperm DNA.

One of the filters was hybridized with probe 1 (1x10⁷ cpm) in 10 ml of the fresh hybridization solution at 30°C for 2 hours. Another filter was hybridized with probe 2 (1x10⁷ cpm) in 10 ml of the fresh hybridization solution at 45°C for 2 hours.

After hybridization, the filters were washed 3 times at 30°C in 6xSSC (0.9 M NaCl, 0.09 M Na-Citrate) for 30 minutes, dried in air and then exposed to X-ray films (XAR-5, Kodak) with intensifying screens (Kodak) at -70°C for 12 hours. 42 colonies showed a positive reaction against both radioactive probes.

Plasmid DNA was isolated by the alkaline method (See Molecular Cloning, A Laboratory Manual, 1982, Eds., Maniatis, T. et al., p90) from one of the positive colonies. The plasmid DNA was digested with HindIII, BglII, ScaI, BamHI, or a combination of two or more of these enzymes and then analyzed by agarose gel electrophoresis. The gel patterns of the digested DNAs were compared to the digested patterns of pT-1 and the comparison indicated that the plasmid analyzed was the desired one (pSVtPA, Fig. 2B).

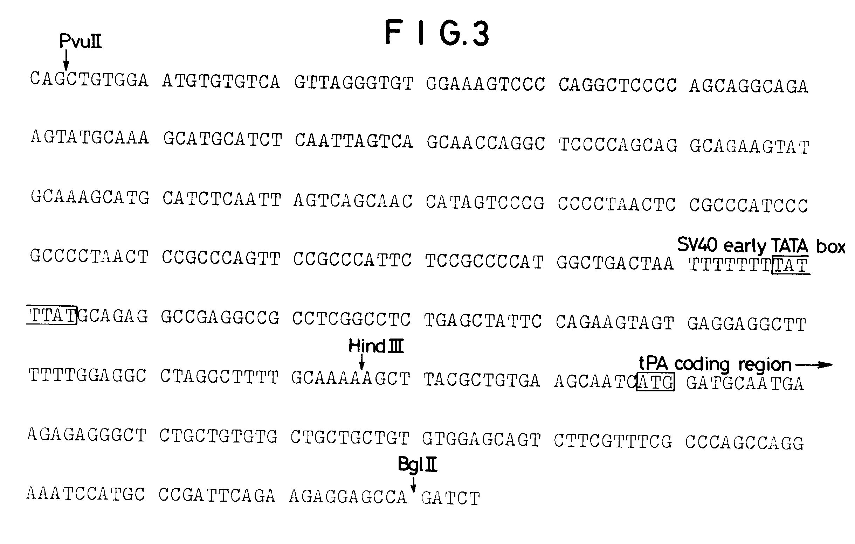

DNA sequences between HindIII and BglII and the upstream of the HindIII site were determined by the chemical method (Maxam, A.M. and Gilbert, W., 1977, Proc. Natl. Acad. Sci., 74:560). Sequence data showed that the plasmid pSVtPA was constructed correctly. Figure 3 provides the DNA sequence between PvuII and BglII of the plasmid pSVtPA. An SV40 early TATA box sequence is indicated in the figure.

### 1-C Construction of Plasmid pHStPA

The 200 bp HindIII fragment obtained in section 1-A contains the hsp83 heat shock regulatory sequences. The HindIII fragment was inserted in the HindIII site of the plasmid pSVtPA obtained in the section 1-B to construct plasmid pHStPA as described below.

pSVtPA was digested with HindIII and treated with BAP. 100 ng of the resulting linear pSVtPA DNA, whose 5'-ends were dephospholylated, and 30 ng of the 200 bp HindIII fragment from section 1-A were dissolved in 19.5 µl of ligation buffer and ligated by adding 0.05 units of T4 DNA ligase and followed by incubation at 15°C for 3 hours. E. coli strain DH-1 was transformed with the ligated DNA sample and ampicillin resistant colonies were obtained.

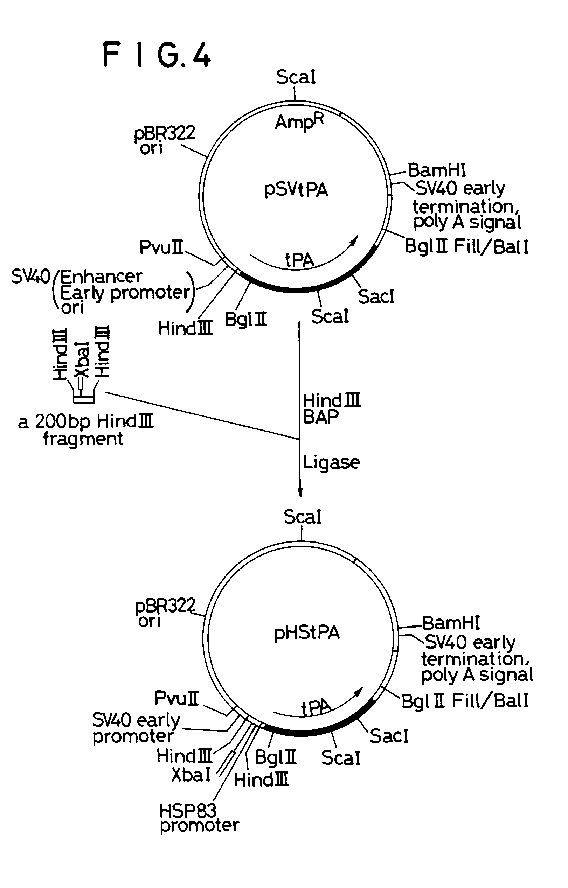

Plasmid DNAs were isolated from 6 colonies by the rapid method (Birnboim, H.C. and Doly, J., 1979, Nucl. Acids Res., 7:1513), digested with the restriction nucleases BglII and Xbal (BAL) and analyzed by polyacrylamide gel electrophoresis. The gel pattern showed that 4 of the plasmids contained the 200 bp HindIII fragment with the correct orientation. Plasmid DNA was purified by the alkaline method from one of the 4 clones. The plasmid DNA was digested with HindIII, BglII, ScaI, BamHI or a combination of two or more of the enzymes and then analyzed by agarose gel electrophoresis. The gel patterns of the digested DNAs showed that the plasmid analyzed was the desired one (pHStPA, Figure 4).

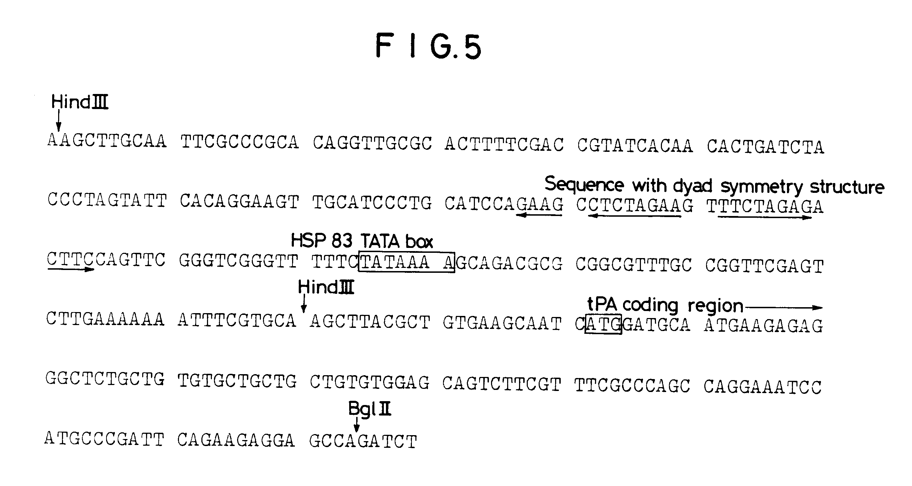

The DNA sequence between XbaI and BglII of the obtained plasmid was determined by the chemical method and the sequence data showed that the plasmid pHStPA was constructed correctly. Figure 5 shows the DNA sequence of the pHStPA DNA fragment which includes two HindIII and one BglII sites. The hsp83 heat shock regulatory sequences are indicated in the figure.

### 1-D Construction of Plasmid pHStPA-tk

A 3.4 kb BamHI fragment containing a herpes simplex virus-1(HSV-1) tk gene (Wigler, M. et al., 1978, Cell, 14:725) was isolated from the plasmid ptk5 (Goodenow, R.S. et al., 1982, Science, 215:677). The 3.4 kb BamHI fragment was inserted in the BamHI site of the plasmid pHStPA (Section 1-C) to construct plasmid pHStPA-tk as described below.

The plasmid pHStPA was digested with BamHI and treated with BAP. 200 ng of the resulting linear pHStPA and 50 ng of the 3.4 kb BamHI fragment were dissolved in 19.5 µl of the ligation buffer and ligated by adding 0.05 units of T4 DNA ligase and followed by incubation at 15°C for 3 hours. E. coli strain DH-1 was transformed with the ligated DNA sample and ampicillin resistant colonies were obtained. Plasmid DNAs were isolated from 7 colonies by the rapid method. The DNAs were digested with BamHI and analyzed with the agarose gel. The gel pattern showed that 6 plasmids contained the 3.4 kb BamHI fragment. These plasmids are designated as pHStPA-tk.

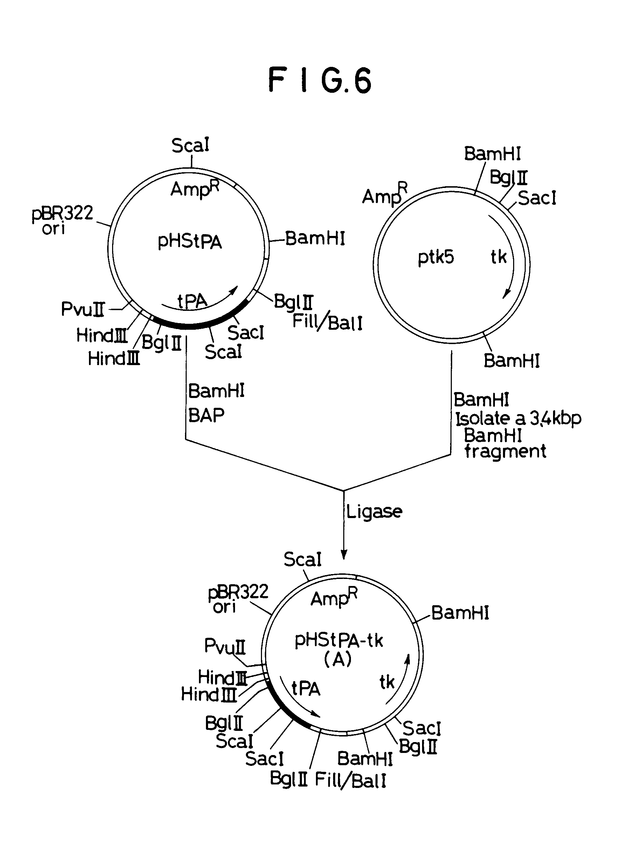

pHStPA-tk was digested with the restriction enzymes BglII and SacI(NEB). Orientation of the inserted DNA was analyzed using the digested DNA fragments by agarose gel electrophoresis The plasmid containing the tPA gene and the tk gene in the same direction was designated as type A. Another plasmid containing two genes in the opposite direction was designated as type B. The type A plasmid (Figure 6) was prepared by the alkaline method.

The 3.4 kb BamHI fragment was inserted in the BamHI site of the plasmid pSVtPA obtained in section 1-B. The resulting plasmid pSVtPA-tk(type A) was used in the control experiments described in the expression experiment of example 2 hereinafter, because this plasmid contained the SV40 early promoter which was connected functionally with the tPA structural gene.

### Example 2

### [Transformation of Mouse L Cells with pHStPA-tk and Regulatory Expression of the tPA Gene in the Transformed Cell.]

The mouse L cell line deficient in the thymidine kinase (tk) activity was prepared and propagated as described by Kit et al. (Kit, S. et al., 1963, Expl. Cell Res., 31:297). The cells were spread at 1x10⁶ cells per 10 cm dish and incubated in RPMI-1640 (Flow laboratories) medium supplemented with 10% fetal calf serum (FCS) at 37°C in an atmosphere of 5% CO₂ for 16 hours.

The cells were transfected with pHStPA-tk (type A) (5 µg DNA per dish) obtained in example 1 by the calcium phosphate method (Graham, F.L. and Van Der Eb, A.J., 1973, Virology, 52:456) and cells were maintained in the MEM (Flow laboratories) medium supplemented with 10% FCS, 5 µg/ml of hypoxanthine, 1 µg/ml of aminopterin and 5 µg/ml of thymidine (HAT medium) for 12 days. Approximately 100 tk positive colonies were obtained from 10 dishes after cultivation in the HAT medium. 96 colonies were isolated using cloning rings and propagated in 24 well dishes containing the HAT medium. When the cells became almost confluent, the medium was aspirated and the RPMI-1640 medium supplemented with no FCS (0% FCS) was added to the cell monolayer. After 24 hours incubation at 37°C in 5% CO₂, an aliquot (20 µl) of each culture medium was picked up and subjected to the measurements of the fibrin-clot lytic activity (Rijken, D.C. et al., 1979, Biochim. Biophys. Acta, 580:140). No activity was detected from the 96 samples when cultured at normal temperature (37°C).

Expression by heat shock induction was then examined and the clones which produce a fibrin-clot lytic enzyme were found among the 96 clones. All the clones were propagated in 24 well dishes. When the cells became to almost confluent, the HAT medium was aspirated and the RPMI-1640 (0% FCS) prewarmed at 43°C was added. The cells were incubated at 43°C in air for 1 hour and then at 37°C in 5% CO₂ for 24 hours. After the 24 hours incubation, aliquot (20 µl) of the culture medium was subjected to the fibrin-clot lytic activity measurement. The measurement indicated that 4 clones secreted a certain fibrin-clot lytic enzyme.

An anti-urokinase did not quench the activity but an anti-human tPA did. The results indicated that the fibrin-clot lytic enzyme was human tPA. The fibrin-clot lytic activity of the secreted tPA was determined by comparison with of that of urokinase. One of the 4 clones, which was designated as #P48, was found to produce tPA at 5.2 units/ml/day. The cells (#P48) was passaged several times for 2 months but the amounts of the secreted tPA did not change during the passages.

In a control experiment, mouse L tk⁻ cells were transfected with pSVtPA-tk by the calcium phosphate method and tk⁺ transformants were selected in the HAT medium. 66 individual colonies were isolated and tPA production by the transformants was measured as described above. 7 clones showed constitutive tPA production under the control of the SV40 early promoter. The strongest tPA producing clone was designated as #P6 and this clone produced tPA at 4.2 units/ml/day.

Since the clone #P48 produced tPA (5.2 units/ml/day) slightly more than the clone #P6 (4.2 units/ml/day) did, strength of the Drosophila hsp83 heat shock promoter was considered to be comparable to that of the SV40 early promoter, which is widely used for expression experiments in mammalian cells.

Heat shock expression by the Drosophila hsp83 heat shock regulatory sequences was further examined using clone #P48.

Amounts of the tPA mRNA of the #P48 cells were measured before or after heat shock induction as follows.

The #P48 cells were grown and subjected to the heat shock treatment (43°C, 1 hour) as mentioned above. After the heat shock treatment, cells were incubated at 37°C in 5% CO₂ for 1, 8, or 24 hours and then harvested. Cells which were not treated at 43°C were also harvested and used for control experiments. Total RNAs were prepared from the harvested cells by the Guanidium-CsCl method (Glisin, V. et al., 1974, Biochemistry, 13:2633). 250-380 µg of total RNAs were recovered from eight 10 cm dishes in each preparation.

Total RNAs were fractionated by the agarose gel containing formamide as reported (Lehrach, H. et al., 1977, Biochemistry, 16:4743), blotted onto nitrocellulose filters (BA85, Schleicher and Schuell) by the Southern method (Southern, E.M., 1975, J. Mol. Biol., 98:503) and baked at 80°C for 2 hours in a vacuum oven. Two series of filters were prepared and used for hybridization experiments.

The filters were prehybridized in 50 ml of the prehybridization solution at 42°C for 4 hours and each filter was hybridized with two different probes at 42°C for 19 hours in 25 ml of the hybridization solution containing 50% formamide. One of the probes was the nick-translated 325 bp PvuII-HindIII fragment (2.6x10⁷ cpm) in which the SV40 early promoter sequence was included (See pHStPA in Fig 4 and pHStPA-tk in Fig. 6) and the other was the nick-translated 470 bp EcoRI fragment (2.6x10⁷ cpm) in which the tPA coding sequence was included. After hybridization, the filters were washed 3 times at 20°C in 2xSSC for 30 minutes, and further 3 times at 20°C in 1xSSC for 30 minutes, then dried in air. The dried filters were exposed to X-ray films with a intensifying screen at -70°C for 12 hours.

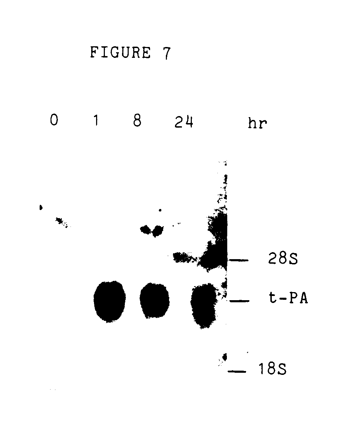

Figure 7 shows the inducible expression (mRNA level) of tPA in the #P48 cells. The nick-translated 470 bp EcoRI fragment hybridized strongly to the certain fraction of the total RNAs obtained from the heat shock treated cells. Number 1, 8, or 24 indicates the incubation time after the heat shock treatment. Number 0 indicates the lane for the total RNAs from the control cells which were not heat treated. Figure 7 shows that the tPA mRNA, which is not detected before the heat shock treatment, is synthesized in large quantity after 1 hour from the heat shock treatment. The amounts of the tPA mRNA decreases during 37°C incubation.

In the #P48 cells, the SV40 early promoter (See pHStPA in Fig 4 and pHStPA-tk in in Fig. 6) instead of the hsp83 heat shock promoter could be considered to drive the transcription of the tPA gene, but the following observations did not support the idea. First, the lane 0 in Fig. 7 showed that the tPA mRNA was not transcribed constitutively under the control of the SV40 promoter. Second, the nick-translated 325 bp PvuII-HindIII probe did not hybridized to any fraction of the total RNAs from the #P48 cells.

Mouse L tk⁺ cells were obtained by transfection of mouse L tk⁻ cells with ptk5 and the total RNAs prepared from the transformed cells were used for the same hybridization experiments as above. The experiments confirmed the absence of tPA mRNA in the mouse L tk⁺ cells before or after heat shock induction.

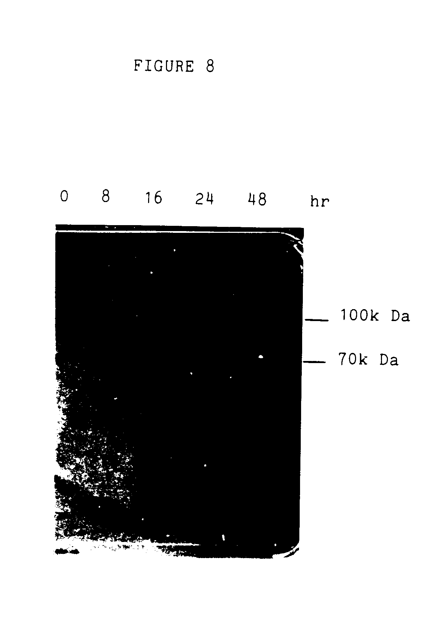

Time course of the tPA accumulation in the culture medium was examined. The #P48 cells were treated at 43°C for 1 hour and then maintained at 37°C in 5% CO₂ for 8, 16, 24 or 48 hours. Measurements of the fibrin-clot lytic activity indicated that the maximum activity appeared after the 16 hours incubation. Proteins in 20 µl of the culture medium obtained before or after the heat shock treatment were separated by 7.5% SDS-polyacrylamide gel electrophoresis (Laemmli, U.K., 1970, Nature, 227:680) and the tPA molecule(s) were detected by the fibrin-agar gel method (Granelli-Piperno, A. and Reich, E., 1978, J. Exp. Med., 148:223). The molecular weight of tPA was calculated to be about 70k daltons as shown in Figure 8. Number 0, 8, 16, 24 or 48 indicates incubation time after the heat shock treatment. The minor band (approx. 100k Da) in Fig. 8 was considered to be the tPA-tPA inhibitor complex as described by Levin (Levin, E.G., 1983, Proc. Natl. Acad. Sci., 80:6804).

While the invention has been explained by a detailed description of certain specific embodiments it is understood that various modifications and substitutions can be made in any of them within the scope of the appended claims which are intended to include equivalents of such embodiments.

## Claims

1. An expression vector capable of inducible expression of a foreign structural gene carried in said expression vector in mammalian host cells which comprises;
a) one or more heat shock regulatory DNA sequences selected from the group consisting of heat shock regulatory DNA sequences from Drosophila melanogaster approximately 83 k daltons heat shock protein (hsp83) gene or modified DNA sequences derived from said heat shock regulatory DNA sequences from said hsp83 gene, by base substitution, deletion, insertion or replacement, which modified DNA sequence has one or more minor nucleotide sequence differences from said heat shock regulatory sequences from said hsp83 gene and at least maintains the efficiency of the heat shock regulatory expression exhibited by the heat shock regulatory sequences of the hsp-83 gene,
b) a desired foreign structural gene which is linked to said one or more heat shock regulatory DNA sequences so as to control the expression of said foreign desired structural gene in host cells by said one or more heat shock regulatory DNA sequences and
c) one or more DNA fragments containing a terminator and a polyadenylation signal.

2. An expression vector claimed in claim 1, wherein said one or more heat shock regulatory DNA sequences is (are) selected from the group consisting of a DNA fragment of approximately 190 base pairs containing heat shock regulatory DNA sequences from Drosophila melanogaster hsp83 gene or modified DNA fragments derived from said DNA fragments of approximately 190 base pairs by base substitution, deletion, insertion or replacement which modified DNA sequence has one or more minor nucleotide sequence differences from said DNA fragments of approximately 190 base pairs and at least maintains the efficiency of the heat shock regulatory expression exhibited by the DNA fragments of approximately 190 base pairs.

3. An expression vector claimed in claim 1, wherein the desired foreign structural gene is originated in eukaryotic cells, and is, for example human tissue plasminogen activator (tPA) gene originated e.g. in normal human cell lines.

4. An expression vector claimed in claim 3, wherein the tPA gene originated in normal human cell lines is human tPA cDNA.

5. An expression vector claimed in claim 1, which further contains a prokaryotic replication origin for replication of said expression vector in host prokaryotic cells.

6. An expression vector claimed in claim 1, which further contains one or more selection markers to select transformed cells by said expression vector, the selection marker being for example ampicillin resistance gene or thymidine kinase gene.

7. An expression vector as claimed in claim 4, which is plasmid pHStPA having the structure shown in Fig. 4, or plasmid pHStPA-tk having the structure shown in Fig. 6.

8. A method for producing desired gene products, which comprises the steps of;
a) introducing into mammalian host cells an expression vector comprising one or more heat shock regulatory DNA sequences selected from the group consisting of heat shock regulatory DNA sequences from Drosophila melanogaster approximately 83 k daltons heat shock protein (hsp83) gene or modified DNA sequences derived from said heat shock regulatory DNA sequences from said hsp83 gene by base substitution, deletion, insertion or replacement, which modified DNA sequence has one or more minor nucleotide sequence differences from said heat shock regulatory sequences from said hsp83 gene and at least maintains the efficiency of the heat shock regulatory expression exhibited by the heat shock regulatory sequences of the hsp83 gene, a desired foreign gene expression of which is controlled by said one or more heat shock regulatory DNA sequences, and one or more DNA fragments containing a terminator and a polyadenylation signal, to obtain transformed cells retaining the introduced expression vectors either which is integrated into chromosome or which replicate outside chromosome,
b) culturing the transformed cells and
c) inducing expression of said desired foreign structural gene carried in the transformed cells.

9. A method claimed in claim 8, wherein the host cell has a character by which the transformed cells can be selected from the host cells into which the expression vector is introduced;
and wherein for example said character is deficiency of thymidine kinase gene and the expression vectors further contain thymidine kinase gene.

10. A method claimed in claim 8, wherein the expression vectors further contain thymidine kinase gene and the host cells are mouse cell strains deficient of thymidine kinase gene.

11. A method claimed in claim 8, wherein the expression vector is plasmid pHStPA having the structure shown in Fig. 4 or pHStPA-tk having the structure shown in Fig. 6.

12. (a) A cell transformed by an expression vector of claim 1; or
(b) a eukaryotic cell transformed by an expression vector of claim 7; or
(c) an animal cell transformed by an expression vector of claim 7.

13. Use of cells according to claim 12, for the manufacture of a medicament.

## Patentansprüche

1. Expressionsvektor, der zu einer induzierbaren Expression eines fremden Strukturgens fähig ist, das in genanntem Expressionsvektor in Säugetier-Wirtszellen vorhanden ist, der umfaßt;
a) eine oder mehrere Hitzeschock-Regulator-DNA-Sequenzen, ausgewählt aus der Gruppe, bestehend aus Hitzeschock-Regulator-DNA-Sequenzen eines annähernd 83 kd großen Heat Shock-Protein-(hsp83)-Gens aus Drosophila melanogaster oder aus modifizierten DNA-Sequenzen, die aus genannten Hitzeschock-Regulator-DNA-Sequenzen von besagtem hsp83-Gen durch Basensubstitution, -deletion, -insertion oder -austausch erhalten wurden, wobei die modifizierte DNA-Sequenz einen oder mehrere kleinere Unterschiede in der Nukleotidsequenz zu den genannten Hitzeschock-Regulator-Sequenzen aus besagtem hsp83-Gen aufweist und zumindest die Effizienz der Hitzeschock-Regulator-Expression, die von den Hitzeschock-Regulator-Sequenzen des hsp83-Gens gezeigt wird, behalten hat,
b) ein erwünschtes fremdes Strukturgen, das an eines oder mehrere der besagten Hitzeschock-Regulator-DNA-Sequenzen gebunden ist, um die Expression des besagten gewünschten fremden Strukturgens in Wirtszellen durch eine oder mehrere der besagten Hitzeschock-Regulator-DNA-Sequenzen zu kontrollieren und
c) ein oder mehrere DNA-Fragmente, die einen Terminator und ein Polyadenylierungssignal besitzen.

2. Expressionsvektor gemäß Anspruch 1, bei dem eine oder mehrere besagte Hitzeschock-Regulator-DNA-Sequenzen ausgewählt wird (werden) aus der Gruppe, bestehend aus einem DNA-Fragment von annähernd 190 Basenpaaren, das Hitzeschock-Regulator-DNA-Sequenzen des hsp83-Gens aus Drosophila melanogaster oder modifizierte DNA-Sequenzen beinhaltet, die aus besagten DNA-Fragmenten mit annähernd 190 Basenpaaren durch Basensubstitution, -deletion, -insertion oder - austausch erhalten wurden, wobei die modifizierte DNA-Sequenz einen oder mehrere kleinere Unterschiede in der Nukleotidsequnz zu den genannten DNA-Fragmenten mit annähernd 190 Basenpaaren aufweist und zumindest die Effizienz der Hitzeschock-Regulator-Expression, die von den DNA-Fragmenten mit annähernd 190 Basenpaaren gezeigt wird, behalten hat.

3. Expressionsvektor gemäß Anspruch 1, bei dem das gewünschte fremde Strukturgen seinen Ursprung in eukaryotischen Zellen hat, und zum Beispiel das humane Gewebs-Plasminogenaktivator (tPA)-Gen ist, das z. Bsp. in normalen menschlichen Zellen vorhanden ist.

4. Expressionsvektor gemäß Anspruch 3, in dem das tPA-Gen, das aus normalen menschlichen Zellen stammt, humane tPA cDNA ist.

5. Expressionsvektor gemäß Anspruch 1, der weiterhin einen prokaryotischen Replikationsstartpunkt für die Replikation des besagten Expressionsvektors in prokaryotischen Wirtszellen enthält.

6. Expressionsvektor gemäß Anspruch 1, der weiterhin einen oder mehrere Selektionsmarker enthält, um transformierte Zellen durch besagten Expressionsvektor zu selektionieren, wobei der Selektionsmarker zum Beispiel das Ampicillin-Resistenzgen oder das Thymidinkinasegen ist.

7. Expressionsvektor, wie in Anspruch 4 beansprucht, der das Plasmid pHStPA mit der Struktur ist, die in Figur 4 gezeigt wird, oder das Plasmid pHStPA-tk mit der Struktur ist, die in Figur 6 gezeigt wird.

8. Verfahren, um gewünschte Genprodukte herzustellen, das folgende Schritte umfaßt;
a) einen Expressionsvektor in Säugetier-Wirtszellen einzuführen, der aus einer oder mehreren Hitzeschock-Regulator-DNA-Sequenzen besteht, ausgewählt aus der Gruppe, bestehend aus Hitzeschock-Regulator-DNA-Sequenzen aus dem annähernd 83 kd großen Hitzeschock-Protein-(hsp83)-Gen aus Drosophila melanogaster oder modifizierten DNA-Sequenzen, die von besagten Hitzeschock-Regulator-DNA-Sequenzen aus besagtem hsp83-Gen durch Basensubstitution, -dletion, -insertion oder austausch erhalten wurden, wobei die modifizierte DNA-Sequenz eine oder mehrere kleinere Unterschiede in der Nukleotidsequenz zu den besagten Hitzeschock-Regulator-Sequenzen aus besagtem hsp83-Gen besitzt und zumindest die Effizienz der Hitzeschock-Regulator-Expression, die von den Hitzeschock-Regulator-Sequenzen des hsp83-Gens gezeigt werden, behalten hat, eine gewünschte Fremdgenexpression, die durch eine oder mehrere der besagten Hitzeschock-Regulator-DNA-Sequenzen kontrolliert wird und ein oder mehrere DNA-Fragmente, die einen Terminator und ein Polyadenylierungssignal enthalten, um transformierte Zellen zu erhalten, die den eingeführten Expressionsvektor, der entweder in ein Chromosome integriert ist oder der außerhalb des Chromosoms repliziert, beibehalten,
b) die transformierten Zellen in Kultur zu halten und
c) die Expression des gewünschten fremden Strukturgens, das in den transformierten Zellen vorliegt, zu induzieren.

9. Verfahren gemäß Anspruch 8, wobei die Wirtszellen eine Eigenschaft besitzen, durch die die transformierten Zellen von den Wirtszellen selektiert werden können, in die der Expressionsvektor eingeführt wurde;
und wobei die besagte Eigenschaft zum Beispiel die Defizienz des Thymidinkinasegens ist und der Expressionsvektor weiterhin das Thymidinkinasegen enthält.

10. Verfahren gemäß Anspruch 8, in der die Expressionsvektoren weiterhin das Thymidinkinasegen enthalten und die Wirtszellen Mäusezellstämme sind, denen das Thymidinkinasegen fehlt.

11. Verfahren gemäß Anspruch 8, bei dem der Expressionsvektor das Plasmid pHStPA mit der Struktur ist, die in Figur 4 gezeigt wird, oder pHStPA-tk mit der Struktur ist, die in Figur 6 gezeigt wird.

12. (a) Eine Zelle, die durch einen Expressionsvektor aus Anspruch 1 transformiert ist; oder
(b) eine eukaryotische Zelle, die durch einen Expressionsvektor aus Anspruch 7 transformiert ist; oder
(c) eine tierische Zelle, die durch einen Expressionsvektor aus Anspruch 7 transformiert ist.

13. Verwendung der Zellen gemäß Anspruch 12 für die Herstellung eines Medikaments.

## Revendications

1. Vecteur d'expression capable d'induire l'expression d'un gène de structure étranger porté par ledit vecteur d'expression dans les cellules d'un mammifère hôte, qui comprend :
a) une ou plusieurs séquences d'ADN régulatrices de choc thermique, choisies dans le groupe constitué par les séquences d'ADN régulatrices de choc thermique du gène protéique (hsp83) de choc thermique, d'environ 83 kDalton, de Drosophila melanogaster ou les séquences d'ADN modifiées dérivées desdites séquences d'ADN régulatrices de choc thermique dudit gène hsp83, par substitution, suppression, insertion ou remplacement de base, laquelle séquence d'ADN modifiée présente une ou plusieurs différences mineures par rapport auxdites séquences d'ADN régulatrices de choc thermique dudit gène hsp83, et au moins maintient l'efficacité de l'expression de régulation de choc thermique présentée par les séquences régulatrices de choc thermique du gène hsp83,
b) un gène de structure étranger cible qui est lié auxdites une ou plusieurs séquences d'ADN régulatrices de choc thermique, de façon à contrôler l'expression dudit gène de structure étranger cible dans les cellules hôtes au moyen desdites une ou plusieurs séquences d'ADN régulatrices de choc thermiques, et
c) un ou plusieurs fragments d'ADN contenant un terminateur et un signal de polyadénylation.

2. Vecteur d'expression selon la revendication 1, dans lequel lesdites une ou plusieurs séquences d'ADN régulatrices de choc thermique est ou sont choisies dans le groupe constitué par un fragment d'ADN d'environ 190 paires de base contenant des séquences d'ADN régulatrices de choc thermique du gène hsp83 de Drosophila melanogaster ou les fragments d'ADN modifiés dérivés desdits fragments d'ADN d'environ 190 paires de base par substitution, suppression, insertion ou remplacement de base, laquelle séquence d'ADN modifiée présente une ou plusieurs différences mineures dans la séquence nucléotide par rapport auxdits fragments d'ADN d'environ 190 paires de base et au moins maintient l'efficacité de l'expression de régulation de choc thermique présentée par les fragments d'ADN d'environ 190 paires de base.

3. Vecteur d'expression selon la revendication 1, dans lequel le gène de structure étranger cible provient de cellules eucaryotiques, et qui est par exemple le gène activateur tissulaire du plasminogène humain (tPA) provenant par exemple de lignées cellulaires humaines normales.

4. Vecteur d'expression selon la revendication 3, dans lequel le gène tPA provenant de lignées cellulaires humaines normales est l'ADNc de tPA humain.

5. Vecteur d'expression selon la revendication 1, qui contient en outre une origine de réplication procaryotique pour la réplication dudit vecteur d'expression dans lesdites cellules procaryotiques hôtes.

6. Vecteur d'expression selon la revendication 1, qui contient en outre un ou plusieurs marqueurs de sélection pour sélectionner des cellules transformées par ledit vecteur d'expression, le marqueur de sélection étant par exemple le gène de résistance à l'ampicilline ou le gène thymidine-kinase.

7. Vecteur d'expression selon la revendication 1, qui est le plasmide pHStPA ayant la structure représentée à la figure 4, ou le plasmide pHStPA-tk ayant la structure représentée à la figure 6.

8. Procédé de préparation de produits de gène cibles, qui comprend les étapes consistant à :
a) introduire dans des cellules hôtes de mammifères une ou plusieurs séquences d'ADN régulatrices de choc thermique, choisies dans le groupe constitué par les séquences d'ADN régulatrices de choc thermique du gène protéique (hsp83) de choc thermique, d'environ 83 kDalton, de Drosophila melanogaster ou les séquences d'ADN modifiées dérivées desdites séquences d'ADN régulatrices de choc thermique dudit gène hsp83, par substitution, suppression, insertion ou remplacement de base, laquelle séquence d'ADN modifiée présente une ou plusieurs différences mineures dans la séquence nucléotide par rapport auxdites séquences régulatrices de choc thermique dudit gène hsp83, et au moins maintient l'efficacité de l'expression de régulation de choc thermique présentée par les séquences régulatrices de choc thermique du gène hsp83, une expression de gène de structure étranger cible qui est contrôlée par lesdites une ou plusieurs séquences d'ADN régulatrices de choc thermique, et un ou plusieurs fragments d'ADN contenant un terminateur et un signal de polyadénylation, pour obtenir des cellules transformées retenant les vecteurs d'expression introduits, dont l'un quelconque est intégré dans le chromosome ou qui se réplique à l'extérieur du chromosome,
b) cultiver les cellules transformées et
c) induire l'expression dudit gène de structure étranger cible porté par les cellules transformées.

9. Procédé selon la revendication 8, dans lequel la cellule hôte présente un caractère permettant de sélectionner les cellules transformées parmi les cellules hôtes dans lesquelles le vecteur d'expression est introduit ;
et dans lequel par exemple ledit caractère est la déficience en gène thymidine-kinase et les vecteurs d'expression contiennent en outre le gène thymidine-kinase.

10. Procédé selon la revendication 8, dans lequel les vecteurs d'expression contiennent en outre le gène thymidine-kinase et les cellules hôtes sont des souches de cellules murines déficientes en gène thymidine-kinase.

11. Procédé selon la revendication 8, dans lequel le vecteur d'expression est le plasmide pHStPA ayant la structure représentée à la figure 4, ou le plasmide pHStPA-tk ayant la structure représentée à la figure 6.

12. (a) Cellule transformée par un vecteur d'expression selon la revendication 1 ; ou
(b) cellule eucaryotique transformée par un vecteur d'expression selon la revendication 7 ; ou
(c) cellule animale transformée par un vecteur d'expression selon la revendication 7.

13. Utilisation de cellules selon la revendication 12, pour la fabrication d'un médicament.
